# EUROPEAN PATENT APPLICATION

(11) **EP 1 262 134 A2**
(43) Date of publication of application: **04.12.2002**
(21) Application number: 02445068.6
(22) Date of filing: 29.05.2002
(51) Int. Cl.: A47K 7/02, A61F 13/15

(54) **Combined disposable washing glove and waste bag for absorbent baby's nappies and incontinence pads**

(30) Priority: 01.06.2001 SE 0101935
(71) Applicant: SCA Hygiene Products AB, 405 03 Göteborg (SE)
(72) Inventor: Samuelsson, Ann, SE-437 32 Lindome (SE); Hansson, Roy, SE-431 50 Mölndal (SE); Kling, Robert, SE-511 63 Skene (SE)
(74) Representative: Romare, Laila Anette

(57) **Abstract**

The invention relates to a washing glove (101) for single usage, comprising a first material piece (102), having a principal extent in one plane, and a second material piece (103), having an extent substantially parallel with the first material piece. The first and second material piece (102, 103) are joined together and form a pocket (110) situated between the first and second material piece (102, 103), which pocket, at least during use, has an opening (111) for introduction of a hand, the material pieces (102, 103) comprising surfaces (117, 119) orientated toward the pocket (110) of the washing glove (101) and opposing surfaces (118, 120) orientated toward the outside of the washing glove (101). The washing glove (101) is of such a size and shape that it can be inverted around a baby's nappy or an incontinence pad, such that those surfaces (117, 119) of the material pieces (102, 103) which are orientated toward the pocket (110) of the washing glove (101) and those surfaces (118, 120) which are orientated toward the outside of the washing glove (101) change position with one another.

## Description

### TECHNICAL FIELD:

The present invention relates to a washing glove for single usage which, after use, is suitable as a waste bag for used absorbent articles such as baby's nappies or incontinence pads intended for adult users. The washing glove is primarily intended for cleaning of body parts which have become soiled by urine, excrement and other types of body secretions associated with the use of absorbent articles.

The washing glove comprises a first material piece, which has a principal extent in one plane, and a second material piece, which has an extent substantially parallel with the first material piece, the first and second material piece being joined together. The washing glove further has a pocket situated between the first and second material piece, which pocket, at least during use, has an opening for introduction of a hand, the material pieces comprising surfaces orientated toward the pocket of the washing glove and opposing surfaces orientated toward the outside of the washing glove.

### BACKGROUND:

By baby's nappies and incontinence pads are meant absorbent articles intended to encompass the lower part of the trunk of a user and comprising a branch section, a front and a rear waist section. When absorbent articles according to the above are changed, it often occurs that both the used absorbent article and the lower abdominal region of the user are soiled by urine, excrement or other body secretions. Impurities on the body of a user must therefore be carefully removed before a new absorbent article is applied to the user. A normally occurring first step in this cleaning procedure is to rough-wipe the soiled lower abdominal region in a suitable manner. Toilet paper is usually used for this initial rough-wipe. Subsequent steps are habitually that the lower abdominal region is washed with soap and water or some other kind of cleansing liquid, the lower abdominal region is wiped dry and, finally, a new baby's nappy or incontinence pad is applied to the user. Sometimes, where there is a minor degree of soiling, the first rough-wipe is unnecessary. Instead, cleaning is conducted with soap and water or some other type of cleansing liquid directly after the removal of the used absorbent article.

Special so-called washing gloves for cleaning of, for example, soiled lower abdominal regions of users of absorbent articles are currently available on the market. A washing glove normally consists of two substantially rectangular material layers, which have been joined together along three edges such that a pocket has been formed between the material layers. Along one edge of the washing glove, the two material layers are not habitually joined together, instead this can be open such that the hand can be introduced into the washing glove when the washing glove is to be used. Washing gloves of the type specified are dimensioned according to an adult's hand size. Examples of a washing glove of the above-specified type are described in patent application WO 96/16217.

One drawback with washing gloves of traditional type is that they are only used for the very brief moment in which the cleaning is in progress. Directly after the conclusion of the cleaning, the washing glove is discarded and thereafter constitutes nothing other than a component which adds to the volume of refuse without providing any further benefit. As mentioned above, another problem with existing washing gloves is that they are dimensioned such that the pocket shall accommodate a normal-sized hand, which means that the available wiping surface (the surface of the outside of the washing glove) is inadequate for cleaning a heavily soiled lower abdominal region of, for example, a person using larger incontinence pads.

Another drawback is that the cost of washing gloves, moreover, has been felt by many people to be far too high in relation to the limited benefit implied by the use of washing gloves.

After a new absorbent article has been applied to a user, the used absorbent article, which can often be heavily soiled with both urine and excrement and can also be extremely foul-smelling, still remains to be taken in hand. Since absorbent articles such as nappies and incontinence pads cannot or must not normally be flushed down in toilets, inter alia because of the risk of blocking the drainage system, the soiled absorbent articles must be taken in hand and disposed of by some alternative method. Instances also arise in which used nappies or incontinence pads have to be transported from the changing site, for example in connection with nappy changes of infants when there is no toilet available, such as, for example, countryside excursions. There are also instances in which it is desirable to take a changed incontinence pad away with one from the place where the changing was performed in order to conceal the existence of an incontinence problem. Even where there are special disposal bins for used nappies and incontinence pads, there may be interest in somehow encapsulating a heavily soiled used nappy or incontinence pad.

What usually occurs is that the absorbent article is initially disposed of in some form of bin or carrier bag placed in the room in which the changing takes place. A number of absorbent articles are habitually collected in the bin before being removed from this initial disposal site and ending up in, for example, the municipal refuse-treatment system. It often happens that this initial storage of the used absorbent articles takes place over several days.

Many carers of children and incontinents, who change nappies or incontinence pads, tend to wrap the nappies or incontinence pads in, for example, toilet paper to prevent the soiled absorbent article from contaminating its environment. The main reason for the said wrapping in toilet paper is often to prevent the carer's own hands from becoming contaminated with, above all, excrement. In the changing of nappies or incontinence pads, especially in public toilets such as, for example, at sports sites or in theatres, nappies and incontinence pads are often wrapped in toilet paper to prevent the nappy or incontinence pad from being visible to subsequent users of the toilet. A change of nappy or incontinence pad with donned rubber gloves to avoid contamination of the carer's own hands is also a common occurrence, especially within the field of incontinence care for the elderly. Another commonly occurring disposal method is to place the absorbent article in a small plastic bag, which is closed before the nappy or incontinence pad is disposed of in the bin or the carrier bag. This method has the advantage that bad odour is also shut out, whilst the greatest drawback is that the consumption of plastics bags is both expensive and heavy on resources. Nowadays, the overuse of plastics materials is also felt to be extremely distasteful to many consumers due to environmental concerns.

In disposing of the baby's nappy or the incontinence pad, many people habitually fold and/or roll up the baby's nappy or the incontinence pad such that the parts which have not become soiled end up facing outward in the folded-together/rolled up absorbent article. Often, an attempt is also made to seal the folded-up absorbent article, using tape, rubber bands or the like.
For certain types of nappies or incontinence pads, waist-fastening members, usually constituted by a tape tab, are often used for this closure of the folded-up/rolled-up nappy or incontinence pad. When a used baby's nappy or incontinence pad is folded up/rolled up according to this principle, it is sometimes very difficult to carry out the folding-up/rolling up such that all the soiled parts of the baby's nappy or incontinence pad end up away from the surface of the baby's nappy or incontinence pad, often there is some soiled region remaining on the outwardly exposed surface.

Nappies and incontinence pads designed such that the waist-fastening members can be used for the above-described closure of a used, folded-up/rolled up baby's nappy or incontinence pad are well-known and are described, for example, in patent US 5,236,429. The closure of folded-up/rolled up nappies and incontinence pads using the bonded waist-fastening members has more recently, however, been made more difficult, since the adhesive on the waist-fastening members of many modern nappies and incontinence pads has been replaced by mechanical waist-fastening members of the velcro type, which cannot be fixed to the plastics film which often constitutes the outwardly exposed surface of a folded-up/rolled-up nappy or incontinence pad.

Separate closing members for a folded-up/rolled-up nappy or incontinence pad are also found, these are especially usable for trouser-type absorbent articles which are provided with a fully encompassing waist part and do not therefore have any waist-fastening members. Such closing members are described, for example, in patent EP 0,321,234.

One-piece bags specially designed for absorbent articles, above all for smaller absorbent products, such as sanitary towels, are commonly found. The sanitary towels are in this case separately packed in these one-piece bags when sold to consumers. These one-piece bags are also often used as waste bags or as wrapping material for used sanitary towels. The used sanitary towel is normally placed in the one-piece bag in which the newly applied new sanitary towel was packed prior to its use. Certain types of one-piece bags are torn apart along their edge seals when the unused sanitary towel is removed from the one-piece packaging, whereupon the one-piece material becomes well-suited to being wrapped around the used towel. The advantage of these one-piece bags is that they have two fields of application, namely, on the one hand, as packaging for the unused sanitary towel up to the point of use and, on the other hand, as a waste bag for the spent sanitary towel. Described one-piece bags cannot, however, be used to wipe soiled body parts, since they are constituted by merely plastics film or by non-absorbent paper. One-piece bags of this type are described, inter alia, in patent EP 0,675,703 (P&G, Fold and wrap package for catamenial pads providing convenient disposal) and in Swedish patent application no. 9902207-1.

One-piece-packed sanitary towels in which a wipe is jointly packed are described in patent application EP 0,637,950 (P&G, Individually packed sanitary napkin having cleansing wipe packaged therewith). The advantage of a product according to this patent is that all the components essential to a product change (cleansing wipe and disposal bag) are included in the product system. One drawback is, however, that each unit of the product system, such as cleansing wipe and disposal bag, are separate products which have been packed together, which can create major problems, not least in the production, where components from various essentially different production machines are to be combined in one and the same product. Another drawback is that, just as in the use of traditional washing gloves, a jointly packed cleaning product is only used for the very brief moment in which the cleaning is in progress. Directly after the conclusion of the cleaning, the cleaning product is discarded and thereafter constitutes a component which merely adds to the volume of refuse without providing any further benefit. A further drawback of a product according to this patent is that the cleansing wipe does not have any arrangement for securing the cleansing wipe during wiping.

One problem with the use of contemporary washing gloves is that the fixing of a washing glove against the hand is poor, owing to the over-large size of the washing glove in relation to the hand. In the production of washing gloves, low production costs are very important, which means, inter alia, that all washing gloves are produced substantially in one size. The principle in the design of contemporary washing gloves is that they must be able to be used by the great majority of the people who have a need for washing gloves, the washing gloves being designed such that the size is also adequate for people having unusually large hands. Since a great majority of the users of washing gloves have substantially smaller hands than the hands for which the washing gloves have been designed, this means that the washing gloves have not been matched to the majority of the users. An over-large washing glove means poor fixing of the washing glove to the hand of the user, the washing glove therefore moves easily relative to the hand of the person performing the wiping, culminating in an unsatisfactory wiping result. Sometimes the washing glove is crumpled up from the inside of the washing glove by the user, who thereby forms a handle inside the washing glove to enable the washing glove to be fixed relative to the hand. The drawback with this process is that very valuable wiping material is used up for the creation of this handle. Wiping cloths comprising fixing members for secure holding of the wiping cloth during wiping are described in Swedish patent applications no. 0003114-6 and no. 0003113-8.

### ACCOUNT OF THE INVENTION

Many problems associated with the changing of baby's nappies or incontinence pads, cleaning associated with the changing and disposal/storage of soiled baby's nappies or incontinence pads, which problems have been described in the above background section, are solved by virtue of the present invention.

A washing glove of the type referred to in the introduction and realized according to the invention is primarily characterized in that the washing glove, after cleaning of the lower abdominal region, can be inverted around a soiled absorbent product, such as a baby's nappy or an incontinence pad, such that those surfaces of the material pieces which are orientated toward the pocket and those surfaces which are orientated toward the outside of the washing glove change position with one another.

As the result of such an arrangement, the washing glove can both be used for wiping and can constitute a waste bag which effectively guards against the soiled baby's nappy or incontinence pad contaminating its environment.

When the washing glove constitutes a waste bag for a used nappy or incontinence pad, the pocket of the washing glove expediently has a minimum width (W) of 8 cm and a minimum depth (D) of 12 cm.

The first material piece and the second material piece can be separate material pieces or can constitute parts of one and the same material piece, which has been folded along a fold line and has subsequently has been joined together forming a pocket. To form a pocket between the two material pieces, the first and the second material piece can be joined together along at least one part of the periphery of at least the one material piece.

The opening into the washing glove can be achieved by the existence of an interruption, at least during use, in the joint between the material pieces. Such an interruption can here be constituted by an openable joint between the first and the second material piece.

Alternatively, the opening into the washing glove can be constituted by a slot in the one material piece. Such a slot can be constituted by a slot, which is openable during use, in the one material piece. Opening capability can be achieved, for example, by perforation or by any other type of material weakening. It is further possible to achieve an openable slot by arranging an openable and/or closable material strip over the slot. Providing the washing glove with an openable or break-open opening ensures that a nappy or incontinence pad supplied in the pocket of the washing glove is clean and hygienic up to the point of use.

Those surfaces of at least one of the first and second material piece respectively which, during wiping, are orientated toward the outside of the washing glove can expediently comprise a liquid-absorbent wipe layer.

At least one of the first and second material piece respectively can also expediently comprise an essentially liquid-tight barrier layer. Such a barrier layer protects the hand of the person performing the wiping with the washing glove. The liquid-tight barrier layer also gives increased protection against foul-smelling odours which normally arise when an absorbent article containing, for example, excrement, is left for a few days in, for example, a bathroom in which baby's nappies, in particular, are changed.

The shape of the washing glove can be varied within wide frameworks, as long as the pocket can be inverted and can accommodate an absorbent product. Accordingly, the washing glove can have an essentially square, rectangular, rhombic, rhomboidic or parallel-trapezoidal shape, the first material piece and the second material piece having an essentially square, rectangular, rhombic, rhomboidic or parallel-trapezoidal shape and having essentially the same extent, the material pieces being joined together along at least two edges.

Further, the washing glove can have a triangular shape, the first material piece and the second material piece having an essentially triangular shape and having essentially the same extent, the material pieces being joined together along two edges.

In order to ensure good securement of the washing glove, this can be provided with a fixing member. An example of such a fixing member is a grip flap projecting from the surface of the pocket. Another suitable fixing member is constituted by a high-friction surface, situated inside the pocket of the washing glove, which prevents the washing glove from slipping in relation to the hand of the user. By arranging a fixing member inside the pocket of the washing glove, for fixing of at least one finger or for the thumb of the user, the washing glove can be better secured during use. Such a characteristic of the washing glove according to the present invention is favourable, since the washing glove, compared with traditional washing gloves, is considerably larger than a normal-sized hand.

The washing glove can further advantageously comprise a gripping member, situated on the washing glove, for securing a used absorbent product as the washing glove is inverted. Such a gripping member can be constituted by a fold in one of the first and second material piece respectively, or by a fold formed at the transition between the first material piece and the second material piece. The gripping member can be constituted by a fold situated on an edge lying opposite to the opening of the washing glove.

By providing the washing glove with a gripping member, a user of the washing glove, having completed the cleaning, can grip the soiled baby's nappy or incontinence pad without the washing glove needing to be removed from the hand and so that the washing glove can be inverted around the soiled baby's nappy or incontinence pad without this needing to be touched in any way other than the contact occurring between the soiled baby's nappy or incontinence pad and the washing glove.

It is further possible for the washing glove to comprise a closing member for sealing of the washing glove after its inversion, when the washing glove contains a used absorbent product such as a used nappy or incontinence pad. The risk of impurities such as urine and excrement from the nappy or incontinence pad stored in the washing glove leaking out from the washing glove is herein eliminated. The sealing member can additionally be designed in such a way that dispersion of bad odour from the nappy is limited or prevented.

A washing glove according to the invention can also be utilized as packaging for an absorbent product, the washing glove, prior to its inversion, being designed to accommodate an unused absorbent product such as an unused nappy or incontinence pad. Customers are thereby offered a complete changing set containing a nappy or an incontinence pad, as well as a washing glove and a waste bag in the form of packaging for the changing set.

The washing glove can be intended to be inverted before the wiping operation, whereupon the liquid-absorbent wipe layer of the washing glove is exposed on the surface of the washing glove.

It is herein possible to provide the wipe layer of the washing glove with some type of active substance, such as disinfectant, skin conditioner, cleansing liquid or the like.

By virtue of the fact that the wiping surfaces of the washing glove are not exposed but are inverted inward before the washing glove is used, the active substance is protected during storage and transport. The wiping surfaces of the washing glove can also have been wettened during the production in order to be usable in places where there is no wiping liquid, for example on journeys, and should not then be exposed during transport and storage.

By virtue of the invention, it is therefore possible to achieve a washing glove with considerably enhanced function and which, after use as a cleaning product, continues to have an important function to fill as a waste bag for the nappy or incontinence pad contaminated by urine and/or excrement.

### BRIEF DESCRIPTION OF THE FIGURES:

The invention will be described in greater detail below, with reference to the illustrative embodiments shown in the appended figures.
- Figure 1: shows a washing glove according to the invention according to a first embodiment.
- Figure 2: shows a washing glove according to the invention according to a second embodiment.
- Figure 3: shows a washing glove according to the invention according to a third embodiment.
- Figure 4: shows a washing glove according to the invention according to a fourth embodiment.
- Figure 5: shows a washing glove according to the invention according to a fifth embodiment.
- Figure 6: shows a washing glove according to the invention according to a sixth embodiment.
- Figure 7: shows a washing glove according to the invention according to a seventh embodiment.
- Figure 8: shows a washing glove according to the invention according to an eighth embodiment.
- Figure 9: shows a washing glove according to the invention according to a ninth embodiment.
- Figure 10: shows a washing glove according to the invention according to a tenth embodiment.
- Figure 11: shows a washing glove according to the invention according to an eleventh embodiment.

- Figure 12: shows a washing glove according to the invention according to a twelfth embodiment.
- Figure 13: shows a section through the washing glove, along the line XIII-XIII, in Figure 12.

### DESCRIPTION OF ILLUSTRATIVE EMBODIMENTS:

The invention relates to a washing glove which can be turned out and in and which is of sufficient size such that, after use, it can be used as a waste bag for the used baby's nappy or incontinence pad from the child or from the adult incontinent.

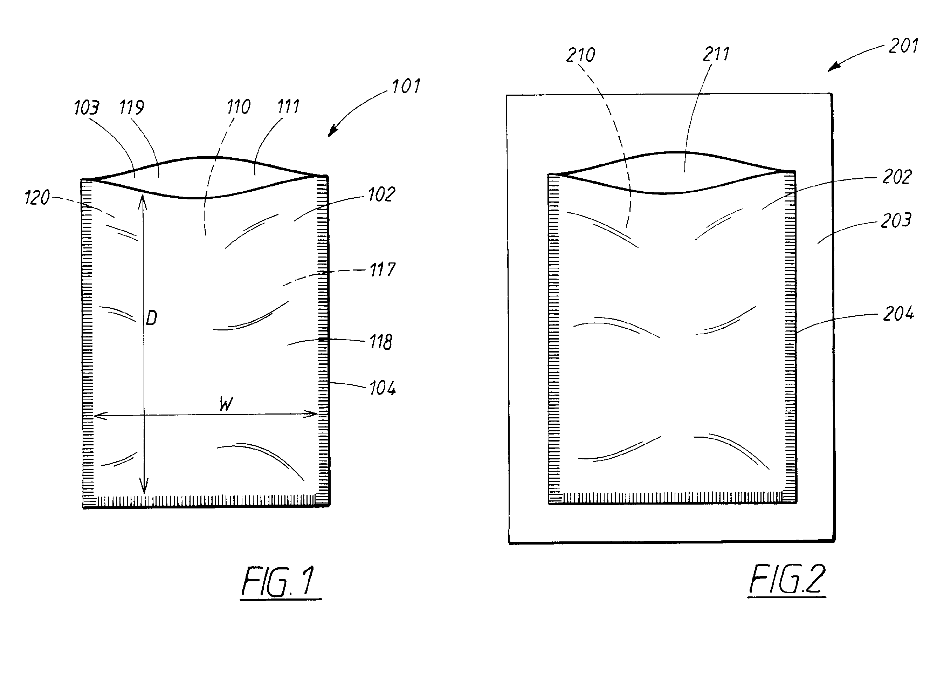

The washing glove 101 shown in Figure 1, intended for single usage, comprises a first material piece 102 and a second material piece 103. The two material pieces 102, 103 are essentially arranged in two mutually adjoining planes. The two material pieces 102, 103 comprise a wipe layer of liquid-absorbent or, at least, hydrophilic material. Suitable materials for the wipe layer are tissue layers, thin, flexible, absorbent foam layers and textile, or textile-like materials, preferably so-called non-woven materials comprising absorbent or, at least, hydrophilic fibres. Examples of usable fibres are cellulose fibres, regenerated cellulose, polyester, hydrophiled polyolefin fibres or the like. The material pieces 102, 103 can comprise absorbent material together with non-absorbent bonding fibres, reinforcing fibres or the like. For example, non-woven materials consisting of fibre mixtures containing absorbent and non-absorbent fibres can be used. It is preferable, however, for the constituent fibres to be substantially made up of fibres which are hydrophilic or which have been treated in order to obtain a hydrophilic surface, for example with wetting agent or by chemical or physical modification of the fibres. The material pieces 102, 103 can comprise one or more material layers and can be provided with casing layers, preferably of non-woven material on the wiping surfaces, being the surfaces which are intended to be on the outside of the washing glove 101, against the surface which is to be wiped.

The first material piece 102 has a first surface 117 arranged against the second material piece 103 and a second surface 118 arranged on the opposite side of the material piece 102. The second material piece 103 has a first surface 119 arranged against the first material piece 102 and a second surface 120 arranged on the opposite side of the material piece 103.

The two material pieces 102, 103 have a rectangular shape with equal length and width proportions and equal-sized surfaces. The two material pieces 102, 103 are placed one against the other without any mutual displacement, the washing glove 101 formed by the two material pieces 102, 103 also being rectangular in shape. The shape of the washing glove and material pieces making up the washing glove can also, of course, be square, rounded or can have shapes which vary within wide limits.

The two material pieces 102, 103 of the washing glove 101 are joined by a glue joint 104 along three of the edges of the washing glove 101. The joint between the material pieces 102, 103 can be realized in a number of alternative ways, examples of alternative connecting methods being heat fusion, ultrasonic welding, stamping, sewing or the like. The joint between the material pieces 102, 103 is expediently realized in such a way that the joint is liquid-tight.

The washing glove 101 has a pocket 110 situated between the two material pieces 102, 103, the first surface 117 of the first material piece 102 and the first surface 119 of the second material piece 103 forming the surfaces of the pocket 110. The second surface 118 of the first material piece 102 and the second surface 120 of the second material piece 103 form the outer limit surfaces of the washing glove 101. The pocket 110 has an opening 111 situated where the material pieces 102, 103 are not joined together.

When the washing glove 101 is used, the hand of the user is introduced through the opening 111 into the pocket 110, after which cleaning/wiping is performed. After completed wiping with the washing glove 101, a folded-up/rolled-up baby's nappy or incontinence pad, situated outside the pocket 110, can be gripped without removal of the hand from the pocket 110 of the washing glove. The washing glove 101 can hereupon be inverted around/turned inside out over the gripped baby's nappy or incontinence pad.
The inversion means that the surfaces of the pocket 110 and the outer limit surfaces of the washing glove 101 change position such that the surfaces 117, 119 initially situated inside the pocket 110, after inversion, constitute environment-facing surfaces and the initially outer limit surfaces 118, 120 of the washing glove 101 constitute surfaces facing the baby's nappy or incontinence pad over which the washing glove 101 has been inverted, the washing glove 101 having become a waste bag for the used baby's nappy or the used incontinence pad.

Inversion according to the method described above thus means that all the surfaces which, after the cleaning, are contaminated by urine and excrement, that is to say the outer limit surfaces 118, 120 of the washing glove 101, together with the baby's nappy or incontinence pad contaminated by urine or excrement, are situated inside the newly formed pocket. At the same time, the surfaces 117, 119 which initially were situated inside the pocket 110 have become outward-orientated surfaces of the combined washing glove 101 and disposal bag. During the whole of the wiping procedure, the surfaces 117, 119 which, after the inversion, are outward-orientated have only been in contact with the hand of the person performing the wiping, an utterly clean and hygienic waste bag having been created by a hygienic inversion operation with minimal risk of contamination of the environment and of the guardian of the urine and excrement, either from the used washing glove 101 or from the used nappy or incontinence pad.

The pocket 110 of the washing glove 101 expediently has a minimum width W of 8 cm and a minimum depth D of 12 cm to enable the washing glove 101 to be inverted around a small baby's nappy. For slightly larger baby's nappies and for smaller incontinence pads, the washing glove needs to have a minimum width W of 14 cm and a minimum depth D of 20 cm. For normal-sized incontinence pads or large baby's nappies, the washing glove should have a minimum width W of 20 cm and a minimum depth D of 30 cm. For the very largest incontinence pads, the washing glove should have a minimum width W of 30 cm and a minimum depth D of 30 cm.

The embodiment shown in Fig. 2 relates to a washing glove 201 which is structured in broadly the same way as the washing glove 101 according to Figure 1.

The washing glove 201 has a first rectangular material piece 202 and a second rectangular material piece 203, the second material piece 203 having a larger extent in the plane than the first material piece 202. All four edges of the first material piece 202 are situated within the edges of the second material piece 203.

The washing glove 201 has a glue joint 204 between the first material piece 202 and second material piece 203 of the washing glove 201. The glue joint 204 follows three of the edges of the first material piece 202. The fourth edge of the first material piece 202, which is unbound to the second material piece 203, constitutes the opening 211 of the washing glove 201 in to the pocket 210 of the washing glove 201. A washing glove according to this embodiment has an enlarged wiping surface, which is especially advantageous when the washing glove 201 is to be used for cleaning heavily soiled adult incontinents.

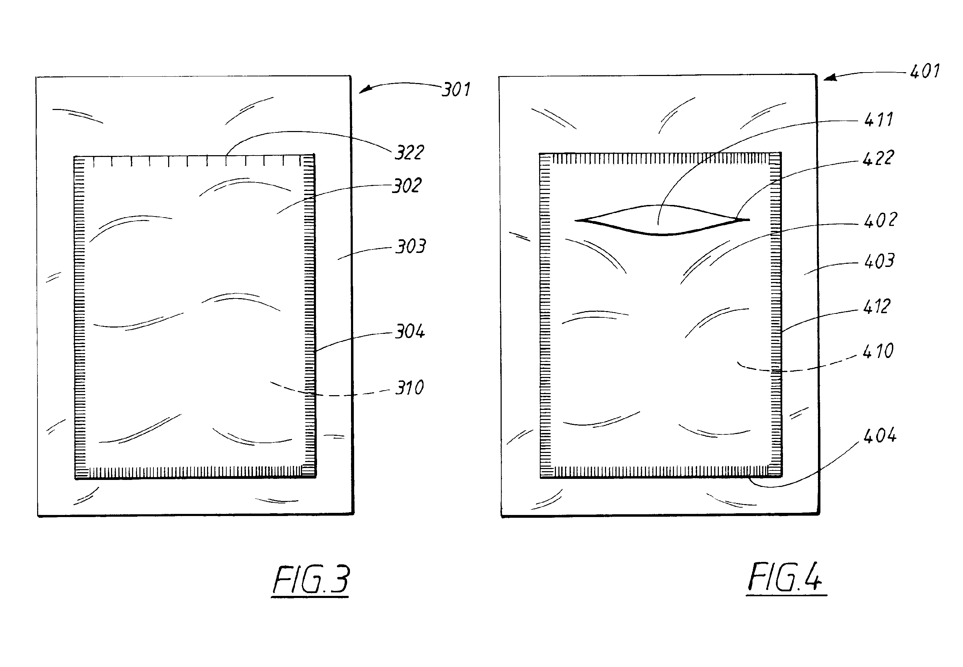

The embodiment shown in Figure 3 relates to a washing glove 301 which is structured in the same way as the washing gloves 101, 201 according to Figures 1 and 2. The washing glove 301 has a first material piece 302 and a second material piece 303.

The first material piece 302 is joined to the second material piece 303 by a permanent glue joint 304 along three of the edges of the first material piece 302 and by a second openable glue joint 322 along the fourth edge. The openable joint 322 can be constituted by a special type of glue or by a substantially reduced quantity of glue per unit area compared with the quantity of glue in which the glue joint 304 is to be permanent. Other special arrangements with respect to an openable glue joint are also conceivable, for example one or both of the surfaces which are to be joined in an openable manner can be treated with release agent. The design of the openable glue joint is not fundamental to this invention. For other connecting methods such as heat fusion, ultrasonic welding, stamping, sewing or the like, the openable joint can also be specially designed, in many connecting methods, for example, the joint is intermittently configured where the joint is to be openable. For certain connecting methods, opening capability can also be achieved by reducing the surface of the joint where it is to be openable, for example the width of the joint can be reduced.

The region within the glue joints 304 and 322 of the washing glove 301 has a pocket 310, which is closed in the production of the washing glove 301. The pocket remains fully sealed right up until the point of use, when the person who is to use the washing glove breaks open the openable joint 322 in order thereafter to use and handle the washing glove 301 in the same way as above-described washing gloves 101, 201 according to the invention. As has been described above, a washing glove 301 according to this embodiment can, for example, contain the new nappy or incontinence pad necessary for the changing.

The embodiment shown in Figure 4 relates to a washing glove 401 having a first material piece 402 and a second material piece 403. The first material piece 402 is joined to the second material piece 403 along the whole of the periphery 412 of the first material piece 402 by means of a glue joint 404. The first material piece 402 here has a slot 422, which constitutes the opening 411 to the pocket 410 of the washing glove 401. The slot 422 can alternatively be sealed at the point of delivery of the washing glove 401 and can be constituted by, for example, an openable perforation, the perforation being torn open in connection with use of the washing glove so that a slot is formed. As has been described above, a washing glove 401 according to this embodiment can, for example, contain the nappy or incontinence pad necessary for the changing.

Washing gloves according to the invention can also be designed such that they have to be inverted prior to wiping, the absorbent wiping material, at the point of delivery, being orientated away from the exposed surface of the washing glove. The wiping surfaces of the washing glove are in this case protected from being dirtied during various types of handling prior to use. The wiping surface of the washing glove can also comprise components which are required to be exposed only in the wiping operation but are otherwise required to be protected from the environment, such as, for example, bacteria-destroying additives. Examples of other additives to the wiping surfaces of a washing glove are various types of ointments for improving the skin condition of the person cleaning. The invertible washing glove can also be wettened during production in order to be usable in places where, for example, there is no water for cleaning, such as on journeys, the wet surface having to be protected such that it does not dry up.

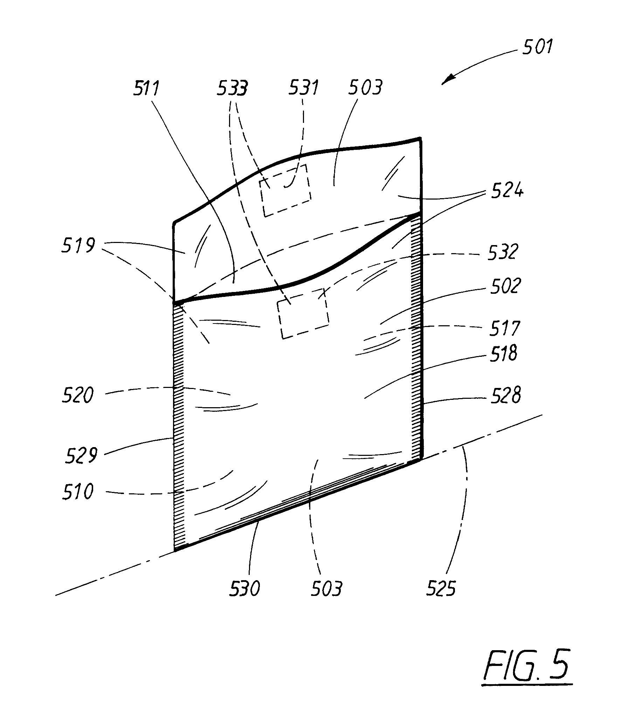

The embodiment shown in Figure 5 relates to a washing glove 501. The washing glove 501 comprises a material piece 524 which is folded around a fold line 525, the washing glove 501 having a folding edge 530. One part of the folded material piece 524 constitutes the first material piece 502 of the washing glove 501 and the remaining part of the folded material piece 524 constitutes the second material piece 503 of the washing glove 501. The washing glove 501 has been folded such that the second material piece 503 extends farther away from the fold line 525 than does the first material piece 502. The fold line 525 can in alternative embodiments be chosen differently, the fold line 525 usually being chosen, for example, such that the first material piece 502 and the second material piece 503 extend equally far away from the fold line 525.

The first material piece 502 has a first surface 517 orientated toward the second material piece 503 and a second surface 518 situated on the opposite side of the first material piece 502. The second material piece 503 has a first surface 519 orientated toward the first material piece 502 and a second surface 520 situated on the opposite side of the second material piece 503. The first material piece 502 and the second material piece 503 are joined by glue joints 528, 529, the washing glove 501 having a pocket 510 having an opening 511.

The washing glove 501 comprises a closing member 533 for closure of the washing glove 501. The closing member 533 comprises a sealing member 531 and a receiving member 532, the closing member 533 being arranged for sealing of the washing glove 501 after inversion, that is to say when the washing glove 501 constitutes a waste bag and contains a soiled nappy or incontinence pad. The closing member 533 is arranged in connection with the opening 510 of the washing glove 501. The sealing member 531 comprises an adhesive layer having good fastening characteristics to the receiving member 532. Other types of closing members can also be used, for example velcro fastenings, buttons and the like.

The closing member 533 in Figure 5 is designed to confine a used nappy placed in the washing glove. Seal-tight closing members for the opening of the washing glove, so that bad odour is prevented from escaping from the washing glove, can also be arranged within the scope of the invention. Closing members can naturally also be arranged for the other described illustrative embodiments.

Washing gloves according to the invention can comprise a liquid-tight barrier layer to protect the hand of the person using the washing glove. The liquid-tight barrier layer is orientated in toward the pocket 510 of the washing glove, that is to say in connection with the first surface 517 of the first material piece 502 and with the first surface 519 of the second material piece 503. The liquid-tight barrier layer can be constituted by a liquid-tight non-woven, a plastics film, or by other types of liquid-tight materials. When the liquid-tight barrier layer is constituted by a plastics film, a comfort-enhancing material can be laminated on the side of the plastics film which is orientated in toward the pocket of the washing glove in order to increase the comfort sensation for the user of the washing glove. The comfort-enhancing material can be constituted by a non-woven material which, in addition to the comfort-enhancing function, also increases the friction against an introduced hand such that the washing glove is better secured during wiping. In order to reduce the use of material in the production of washing gloves comprising liquid-tight barrier layers, the choice can be made to provide one of the first or second material piece of the washing glove with liquid-tight barrier layers as described above. When such a washing glove is used, it is important that the user orientates his/her hand in the pocket of the washing glove such that the liquid-tight barrier layer is situated on the wipe side of the washing glove, that is to say on the inside/palm side of the hand. The liquid-tight barrier layer also means that the function of the washing glove as a waste bag is improved. The waste bag also become liquid-tight, which means that viscous material in the baby's nappy or incontinence pad is prevented from leaking out from the waste bag. A certain odour-reducing effect is also implied by the liquid-tight barrier layer.

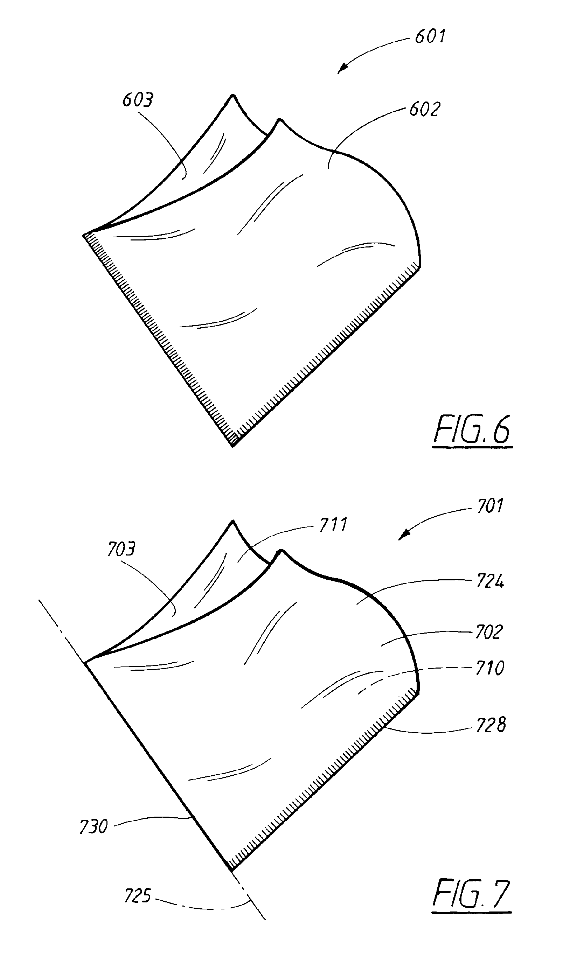

The embodiment shown in Figure 6 relates to a washing glove 601. The washing glove 601 comprises a first material piece 602 and a second material piece 603. The two material pieces 602, 603 are both square in shape and have equal-sized surfaces. The two material pieces 602, 603 are placed one against the other without any mutual displacement, the washing glove 601 formed by the two material pieces 602, 603 also having a square shape. The material pieces 602, 603 are joined together along two neighbouring edges. The washing glove 601 can also, of course, have a square shape, rhombic shape or parallel-trapezoidal shape.

The embodiment shown in Figure 7 relates to a washing glove 701. The washing glove 701 comprises a material piece 724 which is folded around a fold line 725, the washing glove 701 having a folding edge 730. One part of the folded material piece 724 constitutes the first material piece 702 of the washing glove 701 and the remaining part of the folded material piece 724 constitutes the second material piece 703 of the washing glove. The washing glove 701 has been folded such that both the first material piece 702 and the second material piece 703 are square in shape, are uniform and have equal-sized surfaces, the washing glove 701 also having a square shape. The first material piece 702 and the second material piece 703 are joined by a glue joint 728 along one of their edges, the washing glove 701 having a pocket 710 having an opening 711. The washing glove 701 can, of course, also have a square shape, rhombic shape or parallel-trapezoidal shape.

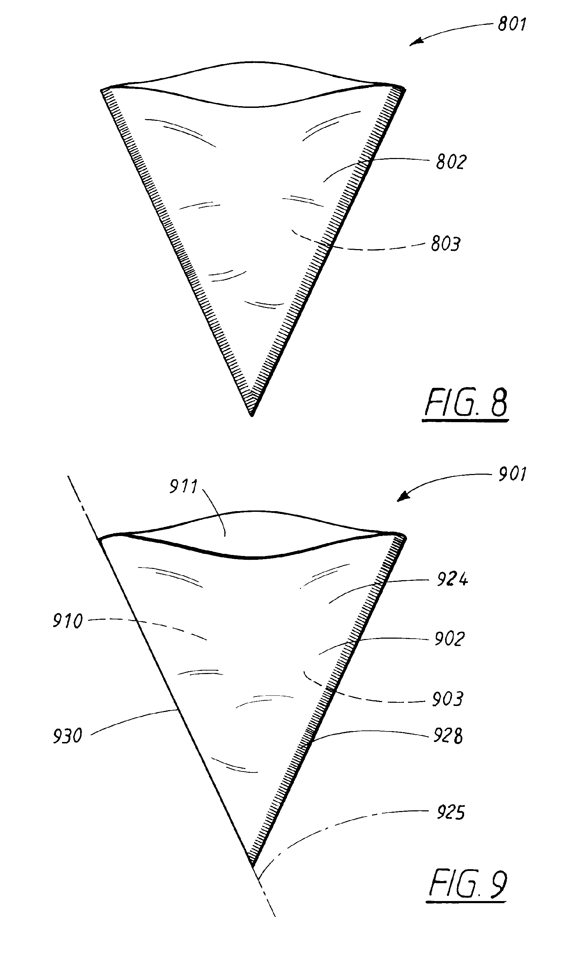

The embodiment shown in Figure 8 relates to a washing glove 801. The washing glove 801 comprises a first material piece 802 and a second material piece 803. The two material pieces 802, 803 are both triangular in shape and have equal-sized surfaces. The two material pieces 802, 803 are placed one against the other without any mutual displacement, the washing glove 801 formed by the two material pieces 802, 803 also having a triangular shape. The material pieces 802, 803 are joined together along two edges.

The embodiment shown in Figure 9 relates to a washing glove 901. The washing glove 901 comprises a material piece 924 which is folded around a fold line 925, the washing glove 901 having a folding edge 930. One part of the folded material piece 924 constitutes the first material piece 902 of the washing glove 901 and the remaining part of the folded material piece 924 constitutes the second material piece 903 of the washing glove. The washing glove 901 has been folded such that both the first material piece 902 and the second material piece 903 are triangular in shape, are uniform and have equal-sized surfaces, the washing glove 901 also having a triangular shape.
The first material piece 902 and the second material piece 903 are joined by a glue joint 928 along one of their edges, the washing glove 901 having a pocket 910 having an opening 911.

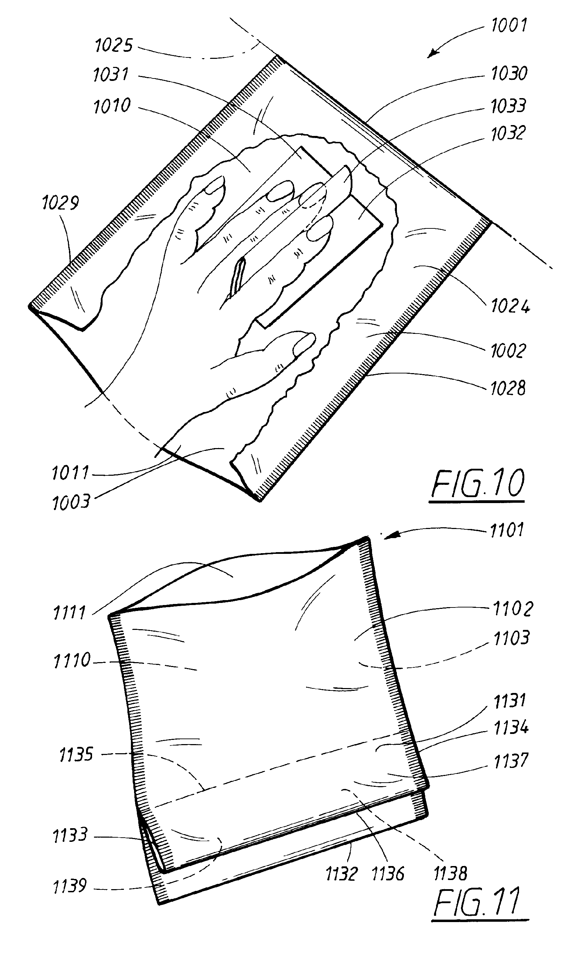

The embodiment shown in Figure 10 relates to a washing glove 1001. The washing glove 1001 comprises a material piece 1024 which is folded around a fold line 1025, the washing glove 1001 having a folding edge 1030. One part of the folded material piece 1024 constitutes the first material piece 1002 of the washing glove 1001 and the remaining part of the folded material piece 1024 constitutes the second material piece 1003 of the washing glove. That part of the first material piece 1002 which normally covers the hand has not been illustrated in Figure 10 for greater clarity.

The washing glove 1001 has been folded such that the second material piece 1003 extends as far away from the fold line 1025 as does the first material piece 1002. The first material piece 1002 and the second material piece 1003 are joined by glue joints 1028, 1029, the washing glove 1001 having a pocket 1010 having an opening 1011. Inside the pocket 1010 of the washing glove 1001, the washing glove 1001 has a fixing member 1031. The fixing member 1031 is formed by an extra material piece 1032, which is fastened to the second material piece 1003 of the washing glove 1001 by means of gluing, welding or the like. The fastening together between the second material piece 1003 of the washing glove 1001 and the extra material piece 1032 can be realized continuously over the whole of the mutually adjoining surfaces of the second material piece of the washing glove 1001 and the extra material piece 1032, or can be in the form of discreet fastening points, or fastening regions disposed between the material pieces 1003, 1032.

The extra material piece 1032 can consist of the same material as the two material pieces 1002, 1003 of the washing glove 1001 or of some other layer material suitable for the purpose. The extra material piece 1032 can be chosen from a wide group of materials, since no absorbency demands are placed upon this extra material piece 1032. Accordingly, the extra material piece 1032 can be a tissue material, a non-woven material, a plastics film material, a paper material or the like. The fixing member 1031 is constituted by a substantially rectangular-shaped grip flap 1033 projecting from the second material piece 1003 of the washing glove 1001 and is formed by a fold, extending in the direction of depth of the pocket 1010, in the extra material piece 1032. The fold can be sealed, for example by gluing or welding, or can be an open material fold. When the washing glove 1001 is used, the fixing member 1031 is secured between two fingers, for example between the middle finger and the index finger, as shown in Figure 10.

Alternative fixing members are also possible, for example, instead of as in the illustrative embodiment according to Figure 10, it is possible to form a fold which is secured between two fingers and to form a tunnel extending in the direction of depth of the pocket 1010, in which tunnel at least one finger is poked for fixing of the washing glove 1001. Examples of fixing members of this type, having a tunnel extending in the direction of depth, are described for a wiping cloth in Swedish patent application no. 0003113-8.

A simple fixing member can consist of a joint, extending in the direction of depth of the pocket 1010, between two material pieces 1002, 1003 of the washing glove 1001, the joint being situated, during use, between two fingers of the hand, for example between the middle finger and the index finger.

Another effective way of arranging better fixing of the washing glove during wiping is to provide the surfaces inside the pocket of the washing glove with a high-friction material. Examples of high-friction material are coarse-fibred non-woven material, plastic mesh, friction glue, foam plastic or the like. The high-friction material can herein constitute the whole of the inside of the pocket or can be arranged on just one part of the inside of the pocket.

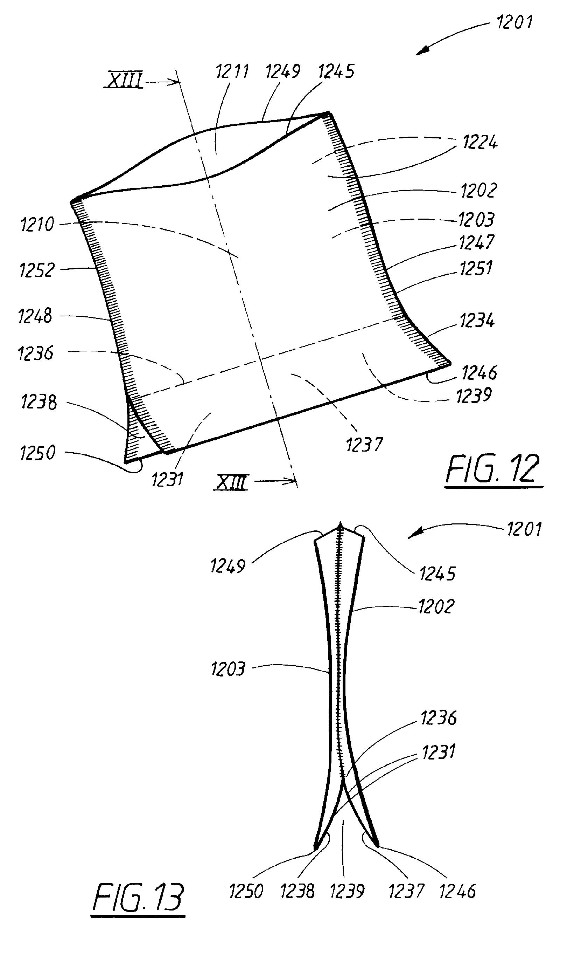

The embodiment shown in Figure 11 shows a washing glove 1101. The washing glove 1101 is rectangular in shape. The washing glove 1101 comprises a first material piece 1102 and a second material piece 1103. The material pieces 1102, 1103 are joined together along three sides, the washing glove having a pocket 1110 having an opening 1111. Close to the bottom 1132 of the pocket, the first material piece 1102 has a fold 1131. The fold 1131 has a longitudinal extent which is parallel with the opening 1111 and bottom 1132 of the washing glove 1101. The fold has an outer fold half 1137 and an inner fold half 1138, the fold halves 1137, 1138 essentially bearing one against the other. The fold 1131 has a base 1135 orientated toward the pocket 1110 of the washing glove and a fold ridge 1136 orientated away from the base, a first end section 1133 and a second end section 1134. In the end sections 1133, 1134 of the fold 1131, the outer fold half 1137 and the inner fold half 1138 are joined together.

The fold 1131 constitutes a gripping member 1139, so that the user of the washing glove 1101, having completed the wiping and without removing the hand from the pocket 1110 of the washing glove, can easily grip a folded-up/rolled-up used baby's nappy or incontinence pad situated outside the pocket 1110. When the user of the washing glove 1101 grips the nappy or incontinence pad, then he/ she expediently introduces his/her thumb into the fold 1131, whilst other fingers remain inside the pocket 1110 of the washing glove 1101 such that the nappy or incontinence pad can be gripped with a stable and secure thumb-fingertip grip. The reverse method, that is to say the thumb of the user inside the pocket 1110 of the washing glove 1101 and other fingers inside the fold 1137 of the washing glove 1101, is an equally good method when a nappy or an incontinence pad has to be gripped.

The washing glove 1101 can subsequently be inverted around/turned inside out over the stably gripped baby's nappy or incontinence pad. This inversion means that the initially inner surfaces of the washing glove 1101 change position such that, after inversion, they constitute environment-facing surfaces, whilst the initially outer surfaces constitute surfaces in toward the baby's nappy or incontinence pad over which the washing glove 1101 has been inverted, the washing glove having become a waste bag for the used baby's nappy or the used incontinence pad.

Inversion of the washing glove 1101, according to the method described above, thus means that all surfaces contaminated by urine and excrement, together with the baby's nappy or incontinence pad contaminated by urine and excrement, are situated inside the newly formed pocket. At the same time, those surfaces which initially were situated inside the pocket 1110 have become outward-orientated surfaces of the washing glove 1101, that is to say the washing glove has been converted into a disposal bag. During the whole of the wiping procedure, those surfaces which are outward-orientated after the inversion have only been in contact with the hand of the person performing the wiping, so that an utterly clean and hygienic waste bag has been created by an utterly hygienic inversion operation with minimal risk of contamination of the environment and of the guardian of the urine and excrement, either from the used washing glove 1101 or from the used nappy or incontinence pad.

The embodiment shown in Figures 12 and 13 shows a washing glove 1201. Figure 13 shows a section through the washing glove 1201 along the line I-I in Figure 12.

The washing glove 1201 comprises a pocket 1210 having an opening 1211. The washing glove 1201 further comprises a gripping member 1239 arranged deep into the pocket 1210 of the washing glove 1201. The washing glove 1201 comprises a material piece 1224, the material piece 1224 comprising a first material piece 1202, a second material piece 1203 and a fold 1231. The first material piece 1202 comprises a first edge 1245, a second edge 1246, a third edge 1247 and a fourth edge 1248. The second material piece 1203 comprises a first edge 1249, a second edge 1250, a third edge 1251 and a fourth edge 1252.

The fold 1231 is arranged at the bottom of the pocket 1210. The fold 1231 comprises a first fold half 1237 and a second fold half 1238. The first fold half 1237 is arranged between the second edge 1246 of the first material piece 1202 and a fold ridge 1236 and the second fold half 1238 is arranged between the second edge 1250 of the second material piece 1203 and the fold ridge 1236. The fold 1231 is orientated into the pocket of the washing glove 1201, the fold ridge 1236 being situated closest to the opening 1211 of the washing glove 1201. The fold 1231 has a longitudinal extent which is parallel with the opening 1211 of the washing glove 1201. In their two end sections 1233, 1234, the first fold half 1237 is joined to the first material piece 1202 and the second fold half 1238 is joined to the second material piece 1203. The two fold halves 1237, 1238 can also be joined together in the end sections 1233, 1234 of the fold 1231. The first material piece 1202 and the second material piece 1203 are joined together along those edges of the washing glove 1201 at right angles to the opening 1211, the joints extending from the opening 1211 to the fold ridge 1236. The joints can also, for alternative embodiments, extend along the whole of those edges of the washing glove 1201 at right angles to the opening 1211.

The fold 1231 constitutes a gripping member 1239, so that the user of the washing glove 1201, having completed the wiping and without removing the hand from the pocket 1210 of the washing glove, can easily grip a folded-up/rolled-up used baby's nappy or incontinence pad situated outside the pocket 1210 and can hereupon obtain a stable and secure thumb-fingertip grip such that a simple and secure inversion of the washing glove 1201 around the used nappy or incontinence pad can be realized.

## Claims

1. Washing glove (101) for single usage, comprising a first material piece (102), which has a principal extent in one plane, and a second material piece (103), which has an extent substantially parallel with the first material piece, the first and second material piece (102, 103) being joined together, having a pocket (110) situated between the first and second material piece (102, 103), which pocket, at least during use, has an opening (111) for introduction of a hand, the material pieces (102, 103) comprising surfaces (117, 119) orientated toward the pocket (110) of the washing glove (101) and opposing surfaces (118, 120) orientated toward the outside of the washing glove (101), **characterized in that** the washing glove (101) can be inverted around an absorbent product such as a baby's nappy or an incontinence pad such that those surfaces (117, 119) of the material pieces (102, 103) which are orientated toward the pocket (110) and those surfaces (118, 120) which are orientated toward the outside of the washing glove (101) change position with one another.

2. Washing glove according to Claim 1, in which the washing glove constitutes a waste bag for a used nappy or incontinence pad, the pocket (110) of the washing glove having a minimum width (W) of 8 cm and a minimum depth (D) of 12 cm.

3. Washing glove according to Claim 1 or 2, in which the first and the second material piece (202, 203) are joined together along at least one part of the periphery of at least the one material piece.

4. Washing glove according to any of the preceding claims, in which the opening (111) is constituted by an interruption, which is arranged, at least during use, in the joint between the material pieces.

5. Washing glove according to Claim 4, in which the interruption in the joint is constituted by an openable joint (322) between the first and the second material piece.

6. Washing glove (401) according to any of Claims 1-3, in which the opening (411) is constituted by a slot (422) in the one material piece.

7. Washing glove according to Claim 6, in which the slot is constituted by a slot, which is openable during use, in the one material piece.

8. Washing glove (101) according to any of the preceding claims, in which those surfaces (118, 120) of at least one of the first and second material piece respectively (102, 103) which, during wiping, are orientated toward the outside of the washing glove (101) comprise a liquid-absorbent wipe layer.

9. Washing glove according to any of the preceding claims, having a folding edge (530), the first material piece (502) and the second material piece (503) being formed by the same material piece (524), which is folded around a fold line (525).

10. Washing glove according to any of the preceding claims, in which at least one of the first and second material piece respectively comprises an essentially liquid-tight barrier layer.

11. Washing glove according to any of the preceding claims, having an essentially square, rectangular, rhombic, rhomboidic or parallel-trapezoidal shape, the first material piece and the second material piece having an essentially square, rectangular, rhombic, rhomboidic or parallel-trapezoidal shape and having essentially the same extent, the material pieces being joined together along at least two edges.

12. Washing glove according to any of Claims 1-10, having an essentially triangular shape, the first material piece (802) and the second material piece (803) having an essentially triangular shape and having essentially the same extent, the material pieces being joined together along two edges.

13. Washing glove according to any of the preceding claims, in which the pocket comprises a fixing member (1031).

14. Washing glove according to Claim 13, in which the fixing member is constituted by a grip flap projecting from the surface of the pocket.

15. Washing glove according to Claim 13, in which the fixing member is constituted by a high-friction surface situated inside the pocket.

16. Washing glove according to any of the preceding claims, in which the washing glove comprises a gripping member (1139), situated on the outside of the washing glove, for securing a used absorbent product as the washing glove is inverted.

17. Washing glove according to Claim 14, in which the gripping member is constituted by a fold (1131) in one of the first and second material piece respectively.

18. Washing glove according to Claim 15, in which the gripping member is constituted by a fold situated on an edge lying opposite to the opening of the washing glove.

19. Washing glove according to any of the preceding claims, in which the washing glove comprises a closing member for sealing of the washing glove containing a used nappy or incontinence pad.

20. Washing glove according to any of the preceding claims, in which the washing glove contains an unused nappy or incontinence pad.

21. Washing glove according to any of Claims 8-20, in which the washing glove is intended to be inverted before the wiping operation, whereupon the liquid-absorbent wipe layer of the washing glove is exposed on the surface of the washing glove.

22. Washing glove according to Claim 21, in which the liquid-absorbent wipe layer of the washing glove comprises disinfectant.

23. Washing glove according to Claim 21, in which the liquid-absorbent wipe layer of the washing glove comprises skin-conditioner.

24. Washing glove according to Claim 21, in which the liquid-absorbent wipe layer of the washing glove is moistened and comprises cleansing liquid.
